# EUROPEAN PATENT APPLICATION

(11) **EP 3 569 175 A1**
(43) Date of publication of application: **20.11.2019**
(21) Application number: 19173623.0
(22) Date of filing: 09.05.2019
(51) Int. Cl.: A61B 18/14

(54) **MEDICAL DEVICE FOR SURGICAL OPERATION**

(30) Priority: 16.05.2018 KR 20180056123
(71) Applicant: Sejong Medical Co., Ltd., Paju-si, Gyeonggi-do (KR)
(72) Inventor: WON, Geun Soup, Paju-si, Gyeonggi-do (KR)
(74) Representative: Balder IP Law, S.L.

(57) **Abstract**

Disclosed is a medical device for surgical operation. The medical device for surgical operation according to the present disclosure includes: a body unit configured to be gripped by an operator and having a supply pipe configured to supply fluid, a suction pipe configured to suck fluid, a regulation unit configured to regulate a flow rate of fluid, and a power connection unit configured to receive applied external power, each of which is installed in the body unit; a surgical treatment unit detachably coupled to the body unit, the surgical treatment unit including a hollow conductive adaptor connected to each of the power connection unit, the supply pipe, and the suction pipe and having an electrode tip protruding at an end thereof and an insertion pipe configured to perform a relative movement in a state of accommodating the adaptor therein and to selectively expose the electrode tip to the outside through the relative movement; and a treatment-switching unit provided in the body unit in a state of being connected to the power connection unit so as to selectively switch between surgical treatments performed by the electrode tip on the affected part. According to the present disclosure, a surgical treatment, such as incision, and a cleansing operation are both performed through the surgical treatment unit formed in a single thin tubular shape. In addition, the electrode tip, which performs a relative rotation with respect to a body unit, is selectively accommodated in the insertion pipe, and the surgical treatment, such as incision, hemostasis, or coagulation of an affected part, is performed easily and freely using the treatment-switching unit. Therefore, it is possible to perform minimally invasive surgery on the affected part in the body easily, rapidly, and precisely without fear of damage or injury to an internal body tissue or an electrical accident caused by the electrode tip.

## Description

### BACKGROUND OF THE INVENTION

### 1. Field of the invention

The present disclosure relates to a medical device for surgical operation, and more particularly, to a medical device for surgical operation, which is capable of performing a surgical treatment such as incision or hemostasis (coagulation) using high-frequency electricity and is also capable of performing cleaning on an affected part before and after the surgical treatment.

### 2. Description of the Prior Art

A medical device, which is referred to as an electrosurgical scalpel or electrosurgical instrument commonly used in surgical operations, is a device that is capable of incising an affected part with minimal or no pain and stopping the bleeding using electrical energy (about 300 kHz to 3 MHz). The use of such an electrosurgical instrument is almost indispensable in modern surgical operations. Thus, it is no exaggeration to say that the development of such an electrosurgical instrument was the first step in the development of medical technology.

Surgical treatments performed using such an electrosurgical instrument may be broadly classified into incision, hemostasis, and coagulation. "Incision" means a treatment to cut a tumor or tissue using a high-frequency power source, "coagulation" means a treatment to fuse an affected or surgically treated part using energy lower than the power applied during the incision, and "hemostasis" refers to an interim treatment performed between incision and coagulation, i.e. a treatment performed in order to quickly prevent bleeding from the affected or surgically treated part.

An operator may perform a surgical treatment using the electrosurgical instrument by varying each of the frequency and the magnitude of control power provided to the electrosurgical instrument from the outside.

Among the prior art related to electrosurgical instruments described above, Korean Patent No. 10-0389006 (registered on June 13, 2003) discloses a technology for an instrument for detecting and treating a bleeding point using compressed air.

According to this prior art, using the compressed air to instantaneously disperse the blood in the affected part, it is very easy to detect a bleeding point, and by configuring an air brush and an electrocautery device in combination, it is possible to rapidly detect and cauterize the bleeding point, which enables a rapid treatment, and to carry out the whole treatment process without the help of an assistant. Further, since the electrosurgical instrument is configured to be capable of injecting and sucking saline solution, it is possible to clean and detect the surgically treated part, and thus it is possible to effectively treat a bleeding point regardless of the amount of bleeding.

However, a bleeding point detection and treatment device proposed in the prior art still requires improvement in the following aspects, and there is a need for continual improvement.

In the proposed embodiments, in the case in which an electrocautery device, a suction guide tube, a saline solution supply conduit, and an air guide tube, which are to come into direct contact with an affected part, are composed of separate tubular bodies, the outer edge of the bleeding point detection and treatment device is large. Thus, it is difficult to apply the device to minimally invasive surgery on an affected part inside the body, such as laparoscopic surgery, due to the structure of the device.

In addition, even if the bleeding point detection and treatment device having the above-described structure is used in minimally invasive surgery, because the electrocautery device and the like (a suction guide tube, a saline solution supply conduit, and an air guide tube) performing the electrical surgical treatment and cleaning on the affected part are completely fixed to a main body to be held by the operator, so that replacement is not possible. Therefore, it is expected that there will be inconvenience to maintenance due to difficulty in cleaning and disinfection for reuse of the electrocautery device and the like after a surgical treatment. Furthermore, there is an inconvenience in surgery in that in order to rotate the electrocautery device (an electrode point) such that the electrocautery device (electrode point) can be accurately positioned at a location of an affected part in the body, the operator has to rotate his/her hand or wrist or the main body itself.

In addition, because electrode points and legs, which are conductive members constituting the electrocautery device, are exposed to the outside, there is the possibility of an electrical accident and damage to an internal body tissue due to sharp ends during the insertion of the electrocautery device into the body. Thus, a structural improvement is needed in order to effectively prevent these problems and to ensure safe use.

### [Prior Art Document]

### [Patent Document]

Korean Patent No. 10-0389006 (registered on June 13, 2003)

### SUMMARY OF THE INVENTION

The present disclosure aims to provide a medical device for surgical operation, in which a handle part to be gripped and manipulated by an operator during minimally invasive surgery on an affected part in a body and a part introduced into the body to be used as a surgical tool are configured to be detachably coupled to each other, thereby improving the convenience in maintenance of each part, an electrical accident and damage or injury to an internal body tissue is prevented during the insertion of the medical device into the body, and surgical treatments such as incision, hemostasis, and coagulation performed on the affected part using an electrode tip can be easily switched.

In view of the foregoing, a medical device for surgical operation according to the present disclosure includes: a body unit configured to be gripped by an operator and having a supply pipe configured to supply fluid, a suction pipe configured to suck fluid, a regulation unit configured to regulate a flow rate of fluid, and a power connection unit configured to receive applied external power, each of which is installed in the body unit; a surgical treatment unit detachably coupled to the body unit and inserted into an affected part in a body, the surgical treatment unit including a hollow conductive adaptor connected to each of the power connection unit, the supply pipe, and the suction pipe and having an electrode tip protruding at an end thereof and an insertion pipe configured to perform a relative movement in a state of accommodating the adaptor therein and to selectively expose the electrode tip to the outside through the relative movement; and a treatment-switching unit provided in the body unit in a state of being connected to the power connection unit so as to selectively switch a surgical treatment performed by the electrode tip on the affected part.

The treatment-switching unit may include a switching module so as to perform a switching operation such that control power corresponding to at least two of hemostasis, coagulation, incision, and stop modes is selected.

The medical device for surgical operation may further include a cutoff switch provided on a side of the body unit in order to cause the control power applied to the switching module to be selectively transferred to the adaptor.

The cutoff switch may include: a contact terminal configured to move back and forth inside the body unit with respect to the adaptor and to be selectively connected to the adaptor; a leaf spring extending from the contact terminal to be connected to the switching module, and exerting elastic force so as to connect the contact terminal to the adaptor; and a push button having a first end protruding to the outside of the body unit and a second end coupled to the contact terminal so as to selectively connect and disconnect the contact terminal and the adaptor.

The regulation unit may include: a pair of buttons provided to be movable upwards and downwards with respect to the body unit; a pair of pressing rods, each having a first end rotatably provided inside the body unit and a second end provided to be in contact with an end of one of the buttons so as to rotate when the one of the buttons moves upwards, wherein the pressing rods compress and block the supply pipe and the suction pipe, respectively; and a pair of springs provided inside the body unit so as to bias the buttons upwards and to urge a compressing and blocking operation of the pressing rods.

The regulation unit may further include a fixedly holding unit provided between the pair of buttons so as to cause at least one of the buttons to be fixed in a pressed state.

The detachable coupling between the body unit and the surgical treatment unit may be performed using a coupling protrusion body and a fastening sleeve. The coupling protrusion body may include a manifold communicating with each of the supply pipe and the suction pipe and a fitting unit provided to protrude in a state of communicating with the manifold, and the fastening sleeve is fitted into the fitting unit in a state of being coupled to the adaptor.

The relative movement of the insertion pipe with respect to the adaptor may be performed through a knob body that accommodates the fastening sleeve such that the knob body is able to perform restricted sliding relative to the fastening sleeve, the knob body being coupled to the insertion pipe.

The adaptor may further include a support member made of an insulating material. When the insertion pipe performs the relative movement such that the electrode tip is exposed, the support member is elastically deformed while being exposed in a direction away from the electrode tip, so that the electrode tip is stably supported at a position spaced apart from the affected part.

According to the present disclosure, a surgical treatment, such as incision, and a cleansing operation are both performed through the surgical treatment unit formed in a single thin tubular shape. In addition, the electrode tip, which performs a relative rotation with respect to a body unit, is selectively accommodated in the insertion pipe, and the surgical treatment, such as incision, hemostasis, or coagulation of an affected part, is performed easily and freely using the treatment-switching unit. Therefore, it is possible to perform minimally invasive surgery on the affected part in the body easily, rapidly, and precisely without fear of damage or injury to an internal body tissue or an electrical accident caused by the electrode tip.

In addition, since the body unit and the surgical treatment unit are configured to be detachably coupled to each other, disinfection, and washing for replacement or reuse can be facilitated, and convenience in maintenance can be ensured.

### BRIEF DESCRIPTION OF THE DRAWINGS

The above and other aspects, features and advantages of the present disclosure will be more apparent from the following detailed description taken in conjunction with the accompanying drawings, in which:
FIGS. 1A and 1B are perspective views illustrating a medical device for surgical operation according to an embodiment of the present disclosure from different directions, respectively;
FIG. 2 shows an exploded perspective view and a partially enlarged view of FIG. 1A;
FIG. 3 illustrates the operating states of a cutoff switch according to an embodiment of the present disclosure, based on cutting lines in FIG. 1B, respectively;
FIGS. 4A and 4B are operational state views illustrating the operation of a regulator according to an embodiment of the present disclosure;
FIGS. 5A and 5B are operational state views illustrating detachable coupling between a body unit and a surgical treatment unit and the relative movement of an insertion pipe according to an embodiment of the present disclosure; and
FIG. 6 is a view illustrating the operation of a support member attached to an adaptor according to a modification of the present disclosure.

### DETAILED DESCRIPTION OF THE EXEMPLARY EMBODIMENTS

Hereinafter, exemplary embodiments of the present disclosure will be described in detail with reference to the accompanying drawings. In the description of the present disclosure, the description of the well-known function or structure will be omitted in order to clear the subject matter of the present disclosure.

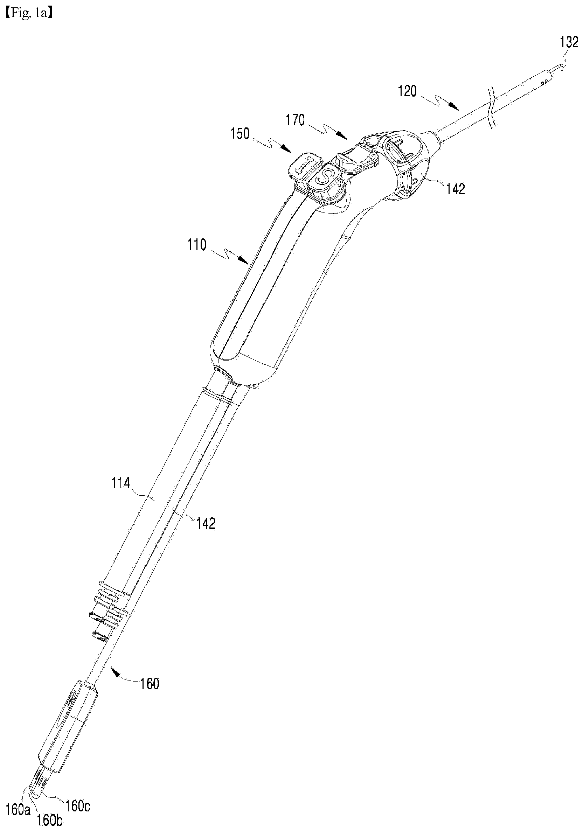

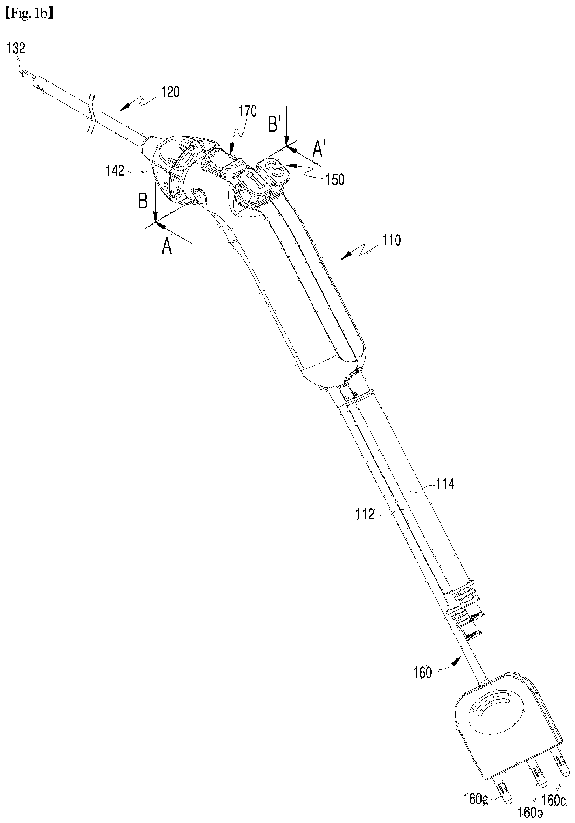

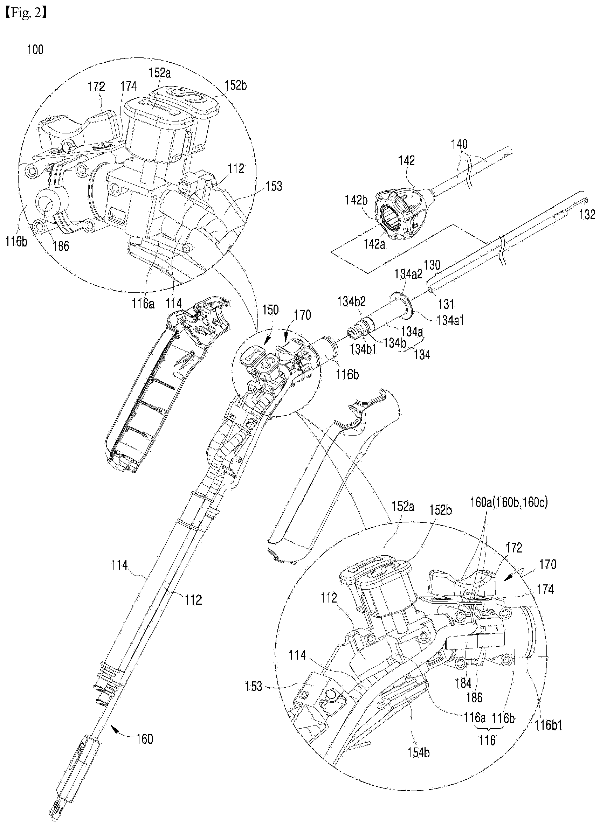

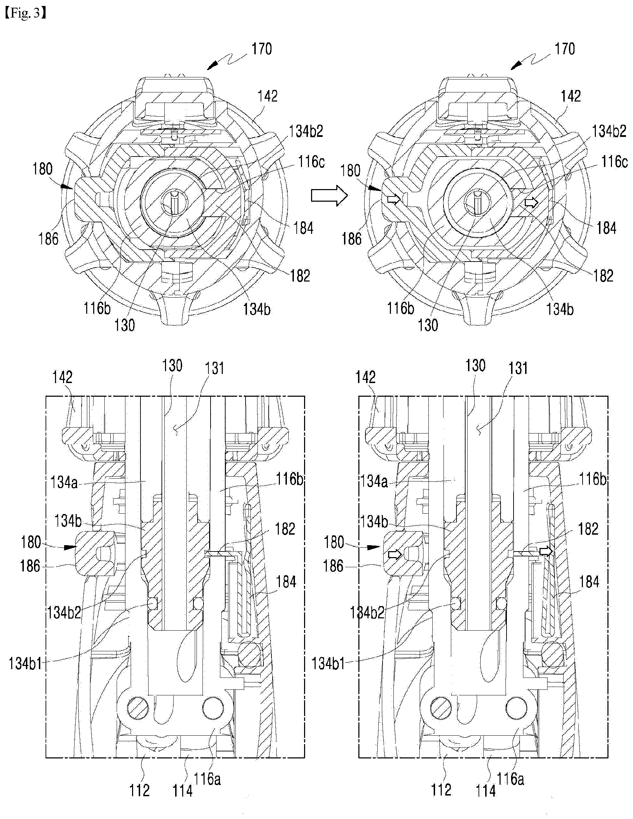

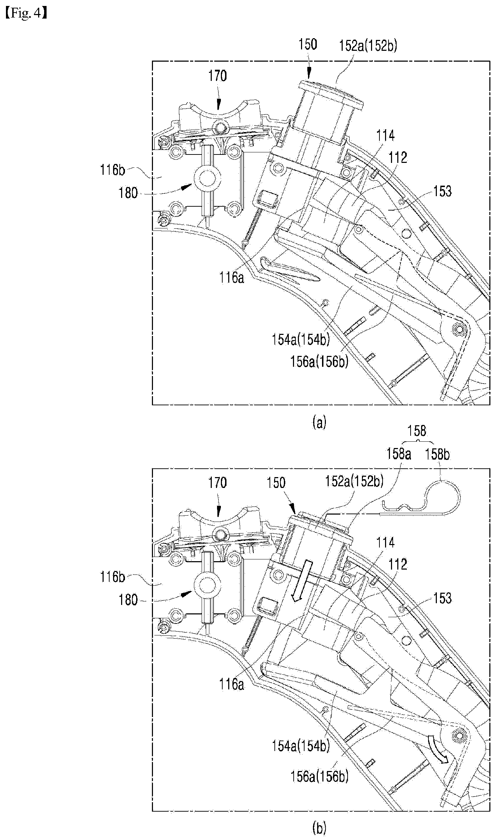

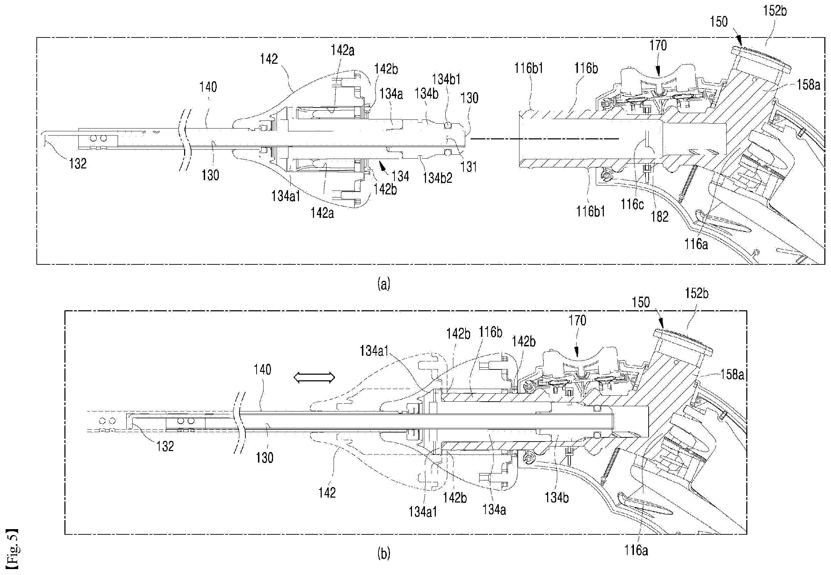

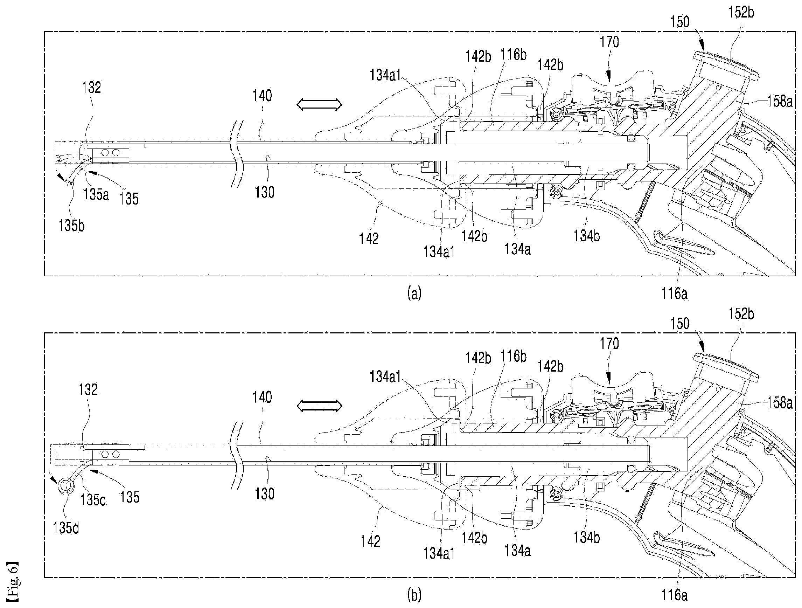

FIGS. 1A and 1B are perspective views illustrating a medical device for surgical operation according to an embodiment of the present disclosure from different directions, respectively, FIG. 2 shows an exploded perspective view and a partially enlarged view of FIG. 1A, FIG. 3 shows views illustrating the operating states of a cutoff switch according to an embodiment of the present disclosure, based on cutting lines in FIG. 1B, respectively, FIGS. 4A and 4B are operational state views illustrating the operation of a regulation unit according to an embodiment of the present disclosure, FIGS. 5A and 5B are operational state views illustrating detachable coupling between a body unit and a surgical treatment unit and the relative movement of an insertion pipe according to an embodiment of the present disclosure, and FIG. 6 is a view illustrating the operation of a support member attached to an adaptor according to a modification of the present disclosure.

"Upper" (upper side), "lower" (lower side), "left", and "right" (side or lateral side), "front" (front side), rear (rear side), and the like designating directions in the description of the disclosure and the claims are determined based on the relative positions between drawings or components for convenience of explanation, rather than limiting the scope of protection. Each of the directions described below is based thereon, unless otherwise specifically limited.

A medical device for surgical operation 100 according to the present disclosure has been conceived in order to ensure ease of application and safety for minimally invasive surgery, the convenience of maintenance of components used in surgery, and convenience, promptness, and preciseness of surgical treatment by an operator, which ultimately leads to rapid treatment and recovery of a patient.

In order to implement the functions and actions described above, the medical device for surgical operation 100 according to the present disclosure may include a body unit 110, a surgical treatment unit 120, a regulation unit 150, a power source connection unit 160, a treatment-switching unit 170, a cutoff switch 180, and the like, as illustrated in the drawings.

Hereinafter, each of the above-described components will be described in detail.

The body unit 110 is a component including a space, in which various components are installed, and gripped by a hand of an operator. In the installation space, a supply pipe 112 and a suction pipe 114 for supplying and sucking a fluid, the regulation unit 150 for regulating the flow rate of the fluid, and the power connection unit 160 for receiving specific control power from the outside may be installed.

The body unit 110 may have any shape, as long as a curved portion, which is able to be comfortably and easily gripped by one hand of an operator and is further able to appropriately hinder the body unit from escaping from the hand in the gripped state, is formed on one side of the outer circumferential surface thereof.

However, as illustrated in FIGS. 1 and 2, the body unit 110 according to an embodiment of the present disclosure may be manufactured in a shape bent at a predetermined angle with respect to the longitudinal direction of the surgical treatment unit 120 to be described later, that is, generally in a pistol shape. This is to prevent the operator's wrist from being excessively bent during surgery inside a body using the surgical treatment unit 120.

A coupling protrusion body 116 is provided inside the body unit 110 having such a shape. The coupling protrusion body 116 is a component that allows each of the supply pipe 112, the suction pipe 114, and the power connection unit 160 to be connected to the surgical treatment unit 120 (specifically, an adaptor 130) to be described later and enables relative rotation and detachable coupling between the surgical treatment unit 120 and the body unit 110.

Specifically, the coupling protrusion body 116 may have an integral structure including a manifold 116a, a fitting unit 116b, and the like, as illustrated in FIGS. 2 to 5.

The manifold 116a is a tubular component having a pair of connection tubes, which respectively communicate with the supply pipe 112 and the suction pipe 114 in the state of being installed in the body unit 110.

In addition, the fitting unit 116b is a component that is formed of a single tubular body. In the state in which the fitting unit 116b is integrated with and communicates with the manifold 116a, the fitting unit 116b is fitted to the surgical treatment unit 120 (specifically, a fastening sleeve 134 of the adaptor 130). The fitting unit 116b includes, on one side thereof, a terminal connection hole 116c formed therethrough in order to electrically connect the adaptor 130 and the power connection unit 160.

As illustrated in FIGS. 3A and 3B, the terminal connection hole 116c allows the control power, which is provided through the treatment-switching unit 170 (a switching module) (and a contact terminal 182 of the cutoff switch 180) connected with the power connection unit 160 (described below), to be applied to the adaptor 130 (specifically, a packing terminal 134b) coupled to the inside of the fitting unit 116b through the fitting unit 116b, which has a tubular shape.

In addition, the terminal connection hole 116c performs a locking function for preventing the surgical treatment portion 120 from being disengaged or separated from the body unit 110 through fitting coupling with the contact terminal 182 of the cutoff switch 180 (described later), and performs an unlocking function for allowing the surgical treatment unit 120 to be disengaged or separated from the body unit 110 through the release of the fitting coupling with the contact terminal 182.

That is, the functions of the terminal connection hole 116c described above are all interlocked with the advancement and retraction of the contact terminal 182 to be described later.

The manifold 116a, the fitting unit 116b, and the terminal connection hole 116c described above may be integrally injection-molded using an insulating engineering plastic material so as to form the coupling protrusion body 116.

The supply pipe 112 is a tubular component configured to receive a fluid supplied from an external fluid supply device (not illustrated), that is, saline solution, an artificial body fluid, air, argon gas, anesthetic gas, or the like, and to supply the fluid to the surgical treatment unit 120.

A first end of the supply pipe 112 may be exposed to the outside of the body unit 110 and communicates with an external fluid supply device (not illustrated), and a second end thereof may be coupled with the manifold 116a of the coupling protrusion body 116 for communication therewith.

The suction pipe 114 is a tubular component for sucking a fluid (saline solution, an artificial body fluid, air, argon gas, or anesthetic gas) that is provided from an external fluid supply device (not illustrated) and then injected into the body for a predetermined purpose through the supply pipe 112 and the surgical treatment unit 120, and for discharging the fluid to the outside.

A first end of the suction pipe 114 may be exposed to the outside of the body unit 110 and communicates with an external fluid supply device (not illustrated) or a storage tank (not illustrated), and a second end thereof may be coupled to communicate with the manifold 116a of the coupling protrusion body 116.

The surgical treatment unit 120 is a component that is inserted into an affected part in the body to perform a surgical treatment, such as incision, hemostasis, or coagulation, and to perform cleaning around the affected part through injection and suction of the fluid provided from the fluid supply device (not illustrated). The surgical treatment unit 120 may be formed in a thin and long tubular shape, and may be detachably coupled to the body unit 110 described above.

In order to perform the functions or actions described above, the surgical treatment unit 120 according to an embodiment of the present disclosure may include the adaptor 130, an insertion pipe 140, and the like, as illustrated in FIGS. 1 to 5.

Specifically, the adaptor 130 is a component that is formed in a tubular shape using a conductive material to allow an surgical treatment, such as incision, hemostasis, or coagulation to be performed by sequentially receiving control power supplied from an external power supply (not illustrated) through the power connection unit 160, the treatment-switching unit 170, and the terminal connection hol3 116c of the fitting unit 116b (to be described later), and making the control power flow to the affected part in the body, and to perform the function of injecting and sucking a fluid by communicating with each of the supply pipe 112 and the suction pipe 114 through a hollow portion 131 formed through the inside thereof.

The adaptor 130 may have an electrode tip for surgical treatment 132 provided at a first end thereof to protrude such that a surgical treatment can be precisely performed with directivity to the affected part through the flow of control power to the affected part in the body, and may be inwardly curved as illustrated in FIGS. 2, 5, and 6.

At this time, the technical content and principle related to a surgical treatment, such as incision or hemostasis (coagulation) performed using selected electric energy (in the range of about 300 kHz to 3 MHz) flowing into the affected part through the conductive adaptor 130 are known in the art, and thus a detailed description thereof will be omitted.

In contrast, at the second end of the adaptor 130 opposite the first end at which the electrode tip 132 is provided, a fastening sleeve 134 may be provided. Here, the fastening sleeve 134 is a component that enables the above-described coupling protrusion body 116 to be detachably coupled to the fitting unit 116b and relative rotation or the like of the surgical treatment unit 120 with respect to the body unit 110 in the state of being integrally coupled so as to accommodate the adaptor 130 therein, and allows the control power to be applied to the adaptor 130 through the terminal connection hole 116c in the fitting unit 116b.

Specifically, the fastening sleeve 134 may include an insulating body 134a, a packing terminal 134b, and the like, as illustrated in FIGS. 3 and 5, in order to implement the above-described functions and actions.

Here, the insulating body 134a is a cylindrical component that is formed of an insulative engineering plastic material and is fitted into the fitting unit 116b so as to accommodate the second end side of the adaptor 130 therein. The insulating body 134a includes a slide plate 134a1 having an end outer edge extended so as to guide sliding thereof with respect to a knob body 142 (described later) while being prevented from being separated by a blocking step 142b of the knob body 142, and a guide groove 134a2 for guiding the sliding of the knob body 142 may be formed in one side of the slide plate 134a1.

The packing terminal 134b is a cylindrical component that is in contact with the insulating body 134a described above, is coupled to accommodate an end of the adaptor 130 therein, and is fitted into the fitting unit 116b in a sealed state. The packing terminal 134b is formed of a conductive material so as to electrically communicate with the adaptor 130, and includes at least one O-ring 134b1 for sealed coupling with the fitting unit 116b on one side of the outer circumferential surface thereof. A ring-shaped fastening groove 134b2, to which a contact terminal 182 (to be described later) is fitted and connected, may be provided around the outer circumference thereof adjacent to the terminal connection hole 116c.

At this time, the O-ring 134b1 performs functions of preventing the fluid bidirectionally flowing through the manifold 116a from leaking and of securely maintaining the coupled state between the fitting unit 116b and the fastening sleeve 134.

Due to the fitting structure between the fastening sleeve 134 integrated with the components described above and the fitting unit 116b of the coupling protrusion body 116, as illustrated in FIG. 5, the surgical treatment unit 120 may be smoothly detachably coupled to the body unit 110 and may rotate relative thereto, and the relative movement of the insertion pipe 140 may be smoothly performed with respect to the adaptor 130.

Meanwhile, as illustrated in FIG. 6, the adaptor 130 according to a modification of the present disclosure may further include a support member 135, which is made of an insulating material and is elastically deformed while being exposed at a position where the support member 135 faces the electrode tip 132 when the insertion pipe 140 is relatively moved (retracted) such that the electrode tip 132 is exposed. The position of the electrode tip 132 can be stably supported to be spaced apart from to an affected part by the support member 135.

The support member 135 described above may be made of acrylonitrile-butadiene-styrene (ABS) resin, which is excellent in processability, moldability, impact resistance, heat resistance, chemical resistance, insulation, and the like, and which can be used in combination with polycarbonate (PC), polyurethane or the like due to excellent compatibility therewith so as to secure required elasticity, or polybutylene terephthalate (PBT) resin, which is excellent in size stability, flame retardancy, insulation, abrasion resistance, and the like, and which can secure necessary elasticity. The support member 135 may be fixedly coupled to the end of the adaptor 130.

More specifically, as illustrated in FIG. 6A, like a bipod that supports a gun barrel on the ground, the support member 135 may include, for example, a pair of abutment rods 135a, the ends of which move away from each other while being elastically deformed in a direction away from the electrode tip 132 when exposed from the insertion pipe 140. At this time, a serrated fixing member 135b for rigidly maintaining a position may be provided at the end of each of the support rods 135a.

The position of the electrode tip 132 is stably supported around an affected part so as to be spaced apart from the affected part through the support member 135 having the structure according to this example (the abutment rods 135a and the fixing members 135b). Therefore, an operator is able to precisely perform a surgical treatment without shaking.

In addition, as another example, as illustrated in FIG. 6B, the support member 135 may include a support rod 135c elastically deformed in a direction away from the electrode tip 132 when exposed from the insertion pipe 140, and a blocking member 135d may be provided at the end of the support rod 135c to close the end of the insertion pipe 140 like a cap when the support rod 135c is accommodated in the insertion pipe 140.

Since the position of the electrode tip 132 is stably supported in the state of being spaced apart from the affected part through the support member 135 (the support rod 135c and the blocking member 135d) having a structure according to another example as described above, the operator is able to precisely perform a surgical treatment on the affected part without shaking, and is also able to completely prevent an electrical accident and damage or injury to an internal body tissue, which may be caused by the electrode tip 132 while the surgical treatment unit 120 is inserted into the body, using the blocking member 135d.

The insertion pipe 140 may be made of a tubular body, which is formed of an insulating material in a structure or a shape that accommodates the above-described adaptor 130 therein so as to be capable of blocking the leakage of control power applied to the adaptor 130.

However, as illustrated in FIGS. 2 and 3B, the insertion pipe 140 according to an embodiment of the present disclosure may be integrally coupled to the knob body 142, which accommodates the fastening sleeve 134 therein so as to be slidable with respect to the fastening sleeve 134 described above.

At this time, the knob body 142 may be formed in a conical shape in which recessed portions, which can be easily gripped by the fingers of an operator, are radially disposed on the outer circumferential surface. Thanks to this shape, it is possible to ensure convenient use by the operator when the surgical treatment unit 120 is rotated relative to the body unit 110.

In addition, as illustrated in FIG. 5, inside the knob body 142, which accommodates the fastening sleeve 134, guide protrusions 142a, which have a cross section having a complementary shape and are fitted into the guide grooves 134a2 formed in the slide plate 134a1 so as to guide the sliding of the knob body 142 and the insertion pipe 140, are provided, so that the relative movement of the insertion pipe 140 with respect to the adaptor 130 can be accurately and smoothly guided.

In addition, as illustrated in FIG. 5, at the end of the knob body 142 that accommodates the fastening sleeve 134, an inwardly protruding blocking step 142b is formed in order to prevent the slide plate 134a1 from being separated, and one or more ring-shaped slide restriction protrusions 1161b1 may be formed to protrude and to be spaced apart from each other on the outer circumferential face of the fitting unit 116b, which comes into contact with the blocking step 142b, to cause the sliding of the knob body 142 and the insertion pipe 140 to be performed stepwise or to restrict the sliding of the knob body 142 and the insertion pipe 140.

Through interaction performed between the knob body 142 integrally coupled to the insertion pipe 140 and the fastening sleeve 134, the electrode tip 132 of the adaptor 130 is able to be precisely accommodated into or drawn out from the insertion pipe 140. Thus, it is possible to fundamentally prevent an electrical accident and damage or injury to by tissue, which may be caused by the sharply protruding electrode tip 132 during the insertion of the surgical treatment unit 120.

As described above, thanks to the surgery treatment unit 120, which is manufactured in a single thin and long dual tubular body shape so as to be detachably coupled to the above-mentioned body unit 110, rotated relative to the body unit 110 and to be selectively inserted into the insertion pipe 140, It is easy to apply the medical device 100 to minimally invasive surgery on an affected part in the body, such as laparoscopic surgery, the medical device 100 can be easily managed, and the convenience of the operation can be improved.

In addition, since various surgical treatments such as incision, hemostasis (coagulation), and the like, and cleaning around an affected part through injection and sucking of a fluid provided from the outside can be performed simultaneously through the surgical treatment unit 120, a precise surgical treatment can be easily performed without any inconvenience.

The regulation unit 150 is a component provided on one side of the supply pipe 112 and the suction pipe 114 described above to regulate the flow rate of the fluid to be injected or sucked. Any regulation unit 150 may be used as long as it has a system or a structure in which one side of the soft supply pipe 112 and the soft suction pipe 114 to allow or interrupt the flow of the fluid so as to regulate the amount of the fluid.

However, as illustrated in FIGS. 2 and 4, the regulation unit 150 according to an embodiment of the present disclosure may include a pair of buttons 152a and 152b, a pair of pressing rods 154a and 154b, and a pair of springs 156a and 156b. These components are provided separately from the above-described coupling protrusion body 116, and may be fastened to the coupling protrusion body 116 or may be mounted on a pipe-mounting bracket 153 integrally formed with the coupling protrusion body 116.

At this time, the pipe-mounting bracket 153 may fixedly support each of the supply pipe 112 and the suction pipe 114 in the state in which the supply pipe 112 and the suction pipe 114 pass therethrough such that the pipe-mounting bracket 153 is stably fastened to the manifold 116a and such that the compressing operation of the pressing rods 154a and 154b, which will be described later, can be stably performed.

The pair of buttons 152a and 152b are components that are provided to be movable upward and downward with respect to the body unit 110 such that external force is directly applied thereon by an operator's finger. Each of the lower ends thereof are brought into contact with an end of one of the pressing rods 154a and 154b (to be described later) configured to compress the lower faces of the supply pipe 112 and the suction pipe 114 to transmit the external force applied by the operator.

The pair of pressing rods 154a and 154b are bar-shaped components provided to selectively compress or decompress the supply pipe 112 and the suction pipe 114, respectively. First ends of the pressing rods 154a and 154b may be rotatably installed inside the body unit 110, more specifically on one side of the pipe-mounting bracket 153, and second ends of the pressing rods 154a and 154b may be provided to be in contact with the lower ends of the buttons 152a and 152b.

At this time, each of the pressing rods 154a and 154b may include a pressing protrusion provided on the central portion thereof to protrude upwards in order to accurately compress one point of a first face of the supply pipe 112 or the suction pipe 114, which is supported by the pipe-mounting bracket 153 on a second face opposite the first face. Thus, the pressing rods 154a and 154b can be rotated in response to the upward and downward movement of the buttons 152a and 152b, thereby accurately compressing and blocking the supply pipe 112 and the suction pipe 114, respectively.

The springs 156a and 156b are a pair of components that exert elastic force in order to bias the buttons 152a and 152b upwards and to perform the operation of compressing and blocking the pressing rods 154a and 154b, respectively. Specifically, the springs 156a and 156b may be implemented using torsion springs 156a and 156b, which are installed between the pressing rods 154a and 154b, each of which is axially coupled to one side of the pipe-mounting bracket 153 to be rotatable, and the pipe-mounting bracket 153 so as to support the pressing rods 154a and 154b to be rotatable upwards.

Meanwhile, as illustrated in FIG. 4, the regulation unit 150 may further include a fixedly holding unit 158 provided between the pair of button units 152a and 152b so as to cause at least one of the button units 152a and 152b to be fixed in the state in which the at least one of the button units 152a and 152b is maintained in the compressed state.

As illustrated in FIGS. 2, 4, and 5, the fixedly holding unit 158 may include a partition wall 158a provided between the button portions 152a and 152b in the form of a thin wall, and a fixing pin 158b, which penetrates the upper end and fixes the pressed button. At this time, the partition wall 158a may be formed integrally with the above-described coupling protrusion body 116.

Since the buttons 152a and 152b are fixed in the pressed state through the fixedly holding unit 158, the opening of the supply pipe 112 and the suction pipe 114 can be maintained. Therefore, after a product is completed, the inside of the pipes can be completely sterilized, and the subsequent packaging operation can be performed in the sterilized state.

The treatment-switching unit 170 is provided in the body 110 in a state of being connected to the power connection unit 160 and is a component for selectively switching the surgical treatment performed on the affected part using the electrode tip 132. With this arrangement, an operator is able to perform an appropriate and rapid surgical treatment using the medical device 100 according to the present disclosure without directly manipulating an external power supply (not illustrated) installed at a remote location.

The treatment-switching unit 170 according to an embodiment of the present disclosure may be implemented with a switching module including a circuit board 174 and a seesaw button 172, as illustrated in FIG. 2, etc.

Here, the circuit board 174 is a component, to which each of electric lines 160a, 160b, and 160c, and the power connection unit 160, to which two or more control powers having electric energies, each of which corresponds to one of hemostasis, coagulation, and incision, are individually applied, is separately connected, and which is electrically connected to the cutoff switch 180 (to be described later) (specifically, a leaf spring 184 side).

The seesaw button 172 is a component that rotates at multiple stages within a predetermined range and electrically connects any one of the connected electric lines 160a, 160b, and 160c (specifically, a contact terminal 182 to be described later) to the adaptor 130.

Through the switching operation of the switching module, control power corresponding to at least two of hemostasis, coagulation, incision, and stop modes may be selected and applied to the adaptor 130.

The cutoff switch 180 is a component that is connected to the switching module described above so as to simultaneously perform a power on/off function of causing applied control power to be selectively transferred to the adaptor 130, a locking function of preventing the surgical treatment unit 120 from being separated from the body unit 110, and an unlocking function of causing the coupled surgical treatment unit 120 to be separated from the body unit 110.

In order to implement the functions or actions as described above, the cutoff switch 180 according to an embodiment of the present disclosure may include a contact terminal 182, a leaf spring 184, a push button 186, and the like, as illustrated in FIGS. 2 and 3.

First, the contact terminal 182 is a conductive component that moves back and forth inside the body 110 with respect to the adaptor 130, and is selectively connected to the adaptor 130. The contact terminal 182 is provided to protrude inwards from one side of the push button 186 (to be described later) such that the contact terminal 182 performs forward and backward movement through the terminal connection hole 116c of the fitting unit 116b depending on the external force applied to the push button 186.

As illustrated in FIG. 3A, when no external force is applied to the push button 186 in the state in which the conductive packing terminal 134b coupled to the adaptor 130 is fully fitted to the fitting unit 116b, the elastic force of the leaf spring 184 (to be described later) is applied to the contact terminal 182. As a result, the contact terminal 182 is in a forced connection or energization state with the packing terminal 134b through the terminal connection hole 116c.

Thus, the control power applied to the treatment-switching unit 170 (switching module) is consequently applied to the electrode tip 132 via the contact terminal 182, the packing terminal 134b, and the adaptor 130, so that a selected surgical treatment can be performed by the treatment-switching unit 170.

In addition, since the contact terminal 182 is in the state of being fitted into the fastening groove 134b2 in the packing terminal 134b and thus the locked state in which the separation of the surgical treatment unit 120 from the body unit 110 is prevented is maintained, the surgical treatment is performed stably.

In contrast, as illustrated in FIG. 3B, when external force is applied to the push button 186 in the state in which the conductive packing terminal 134b coupled with the adaptor 130 is fully fitted into the fitting unit 116b, the leaf spring 184 (to be described later) is pushed while being elastically deformed by external force. Thus, the contact terminal 182 is disconnected from the packing terminal 134b, and thus the surgical treatment selected by the treatment-switching unit 170 is stopped.

In addition, the surgical treatment unit 120 is placed in the unlocked state in which the surgical treatment unit 120 is separable from the body unit 110 when the contact terminal 182 escapes from the fastening groove 134b2 in the packing terminal 134b. Thus, replacement of the surgical treatment unit 120 can be easily performed.

The leaf spring 184 is a conductive component that extends from the contact terminal 182 to be connected to the switching module and exerts elastic force such that the contact terminal 182 is connected to the adaptor 130. The leaf spring 184 is formed generally in a "⊏" shape such that, in the state in which a first end thereof is connected to the contact terminal 182, a second end thereof will exert elastic force in the state of being supported on the inner face of the body unit 110.

The push button 186 is a component that selectively connects/disconnects the contact terminal 182 protruding inward from one side and the adaptor 130. The push button 186 is provided in a ring shape surrounding the outer circumferential face of the fitting unit 116b such that a first end of the push button 186 protrudes to the outside of the body 110 and a second end of the push button 186 is coupled to the contact terminal 182.

The risk of an electric accident and the like can be further reduced through the power on/off function by the cutoff switch 180 having the above structure, and the unintentional separation of the surgical treatment unit 120 during operation can be prevented. Therefore, an operator is able to perform a surgical treatment more stably.

Although the specific embodiment of the present disclosure has been described above, it is apparent to those skilled in the art that the present disclosure is not limited to the embodiment disclosed herein and various modifications and changes can be made without departing from the spirit and scope of the present disclosure. Therefore, such modifications and changes should not be individually construed from the spirit or point of view of the present disclosure, and it should be understood that modified embodiments belong to the claims of the present disclosure.

### Description of Reference Numerals

100: medical device for surgical operation, 110: body unit
112: supply pipe, 114: suction pipe
116: coupling protrusion body, 116a: manifold
116b: fitting unit, 116b1: slide restriction protrusion
116c: terminal connection hole, 120: surgical treatment unit
130: adaptor, 131: hollow portion
132: electrode tip, 134: fastening sleeve
134a: insulating body, 134a1: slide plate
134a2: guide groove, 134b: packing terminal
134b1: O-ring, 134b2: fastening groove
135: support member, 135a: abutment rod
135b: fixing member, 135c: support rod
135d: blocking member, 140: insertion pipe
142: knob body, 142a: guide protrusion
142b: blocking step, 150: regulation unit
152a, 152b: button, 153: pipe-mounting bracket
154a, 154b: pressing rod, 156a, 156b: spring
158: fixedly holding unit, 158a: partition wall
158b: fixing pin, 160: power connection unit
160a, 160b, 160c: first, second, and third electric lines, 170: treatment-switching unit (switching module)
172: seesaw button, 174: circuit board
180: cutoff switch, 182: contact terminal
184: leaf spring, 186: push button

## Claims

1. A medical device for surgical operation, the medical device comprising:
a body unit configured to be gripped by an operator and having a supply pipe configured to supply fluid, a suction pipe configured to suck fluid, a regulation unit configured to regulate a flow rate of fluid, and a power connection unit configured to receive applied external power, each of which is installed in the body unit;
a surgical treatment unit detachably coupled to the body unit and inserted into an affected part in a body, the surgical treatment unit including a hollow conductive adaptor connected to each of the power connection unit, the supply pipe, and the suction pipe and having an electrode tip protruding at an end thereof and an insertion pipe configured to perform a relative movement in a state of accommodating the adaptor therein and to selectively expose the electrode tip to the outside through the relative movement; and
a treatment-switching unit provided in the body unit in a state of being connected to the power connection unit so as to selectively switch a surgical treatment performed by the electrode tip on the affected part.

2. The medical device of claim 1, wherein the treatment-switching unit includes a switching module so as to perform a switching operation such that control power corresponding to at least two of hemostasis, coagulation, incision, and stop modes is selected.

3. The medial device of claim 2, further comprising:
a cutoff switch provided on a side of the body unit in order to cause the control power applied to the switching module to be selectively transferred to the adaptor.

4. The medical device of claim 3, wherein the cutoff switch includes:
a contact terminal configured to move back and forth inside the body unit with respect to the adaptor and to be selectively connected to the adaptor;
a leaf spring extending from the contact terminal to be connected to the switching module, and exerting elastic force so as to connect the contact terminal to the adaptor; and
a push button having a first end protruding to an outside of the body unit and a second end coupled to the contact terminal so as to selectively connect and disconnect the contact terminal and the adaptor.

5. The medical device of claim 2, wherein the regulation unit includes:
a pair of buttons provided to be movable upwards and downwards with respect to the body unit;
a pair of pressing rods, each having a first end rotatably provided inside the body unit and a second end provided to be in contact with an end of one of the buttons so as to rotate when the one of the buttons moves upwards, wherein the pressing rods compress and block the supply pipe and the suction pipe, respectively; and
a pair of springs provided inside the body unit so as to bias the buttons upwards and to urge a compressing and blocking operation of the pressing rods.

6. The medical device of claim 5, wherein the regulation unit further includes a fixedly holding unit provided between the pair of buttons so as to cause at least one of the buttons to be fixed in a pressed state.

7. The medical device of claim 2, wherein the detachable coupling between the body unit and the surgical treatment unit is performed through a coupling protrusion body and a fastening sleeve, wherein the coupling protrusion body is installed in the body unit and includes a manifold communicating with each of the supply pipe and the suction pipe and a fitting unit provided to protrude in a state of communicating with the manifold, and the fastening sleeve is fitted into the fitting unit in a state of being coupled to the adaptor.

8. The medical device of claim 7, wherein the relative movement of the insertion pipe with respect to the adaptor is performed through a knob body that accommodates the fastening sleeve such that the knob body is able to perform restricted sliding relative to the fastening sleeve, the knob body being coupled to the insertion pipe.

9. The medical device of claim 2, wherein the adaptor further includes a support member made of an insulating material, and
when the insertion pipe performs the relative movement such that the electrode tip is exposed, the support member is elastically deformed while being exposed in a direction away from the electrode tip, so that the electrode tip is stably supported at a position spaced apart from the affected part.
